# EUROPEAN PATENT SPECIFICATION

(11) **EP 1 779 117 B1**
(45) Date of publication and mention of the grant of the patent: **19.05.2010**
(21) Application number: 04778605.8
(22) Date of filing: 19.07.2004
(51) Int. Cl.: G01N 33/58, G01N 33/60, C12Q 1/37

(54) **METHODS FOR MEASURING ADAMTS13 ACTIVITY ON THE SURFACE OF PLATELETS**
VERFAHREN ZUR MESSUNG VON ADAMTS13-AKTIVITÄT AUF DER BLUTPLÄTTCHENOBERFLÄCHE.
PROCÉDÉS POUR MESURER L'ACTIVITÉ DE ADAMTS13 PRÉSENTE À LA SURFACE DES PLAQUETTES

(43) Date of publication of application: 02.05.2007
(73) Proprietor: American Diagnostica Inc., Stamford, CT 06911-0215 (US)
(72) Inventor: GREENFIELD, Robert, S., Trumbull, CT 06611 (US); RANZURMAL, Safi, Trumbull, CT 06460 (US)
(74) Representative: Goddar, Heinz J.
(86) International application number: PCT/US2004/023177
(87) International publication number: WO 2006/019379

(56) References cited:
- WO-A-02/10437
- WO-A-2004/035778
- WO-A1-2004/035778
- US-A1- 2002 136 713
- US-A1- 2003 083 231
- US-A1- 2004 138 187
- SUZUKI M ET AL: "Detection of von Willebrand factor-cleaving protease (ADAMTS-13) in human platelets" BIOCHEMICAL AND BIOPHYSICAL RESEARCH COMMUNICATIONS, ACADEMIC PRESS INC. ORLANDO, FL, US, vol. 313, no. 1, 2 January 2004 (2004-01-02), pages 212-216, XP004479138 ISSN: 0006-291X
- LOTTENBERG R. ET AL: 'Assay of Coagulation Proteases Using Peptide Chromogenic and Fluorogenic Substrates' METHODS ENZYMOL. vol. 80, pages 341 - 361, XP008050335

## Description

### FIELD OF THE INVENTION

The invention provides assays for measurement of ADAMTS 13 activity and diagnosis of thrombotic thrombocytopenia purpura.

### BACKGROUND OF THE INVENTION

Thrombotic Thrombocytopenia Purpura (TTP) is a life threatening thrombotic microangiopathic disease characterized by hemolytic anemia and thrombocytopenia associated with platelet aggregation. The cause of TTP has been recently linked to abnormalities in a metalloproteinase called ADAMTS 13 or von Willebrand factor cleaving protease. ADAMTS 13 is an enzyme that is present in significant levels in plasma, and may be expressed in other tissues (Levy et al. 2001, Nature 413:488-494; Plaimauer et al. 2002, Blood 100:3626-3632). Suzuki et al. (2004, Biochem. Biophys. Res. Com. 313 :212-216) recently reported ADAMTS 13 in platelets.

ADAMTS 13 functions by cleaving ultralarge von Willebrand factor (VWF) multimers to smaller VWF proteins. Decreased VWF cleaving protease activity leads to persistence of unusually large multimers of VWF that bind to platelets, causing platelet aggregates, microangiopathic hemolysis, and thrombocytopenia in patients with TTP. Clinical manifestations of TTP are difficult to distinguish from hemolytic uremic syndrome (HUS), another thrombotic microangiopathic disorder. Recent studies indicate that low levels of ADAMTS13 activity are associated with TTP, but not HUS (Veyradier A, et al. 2002, Blood 98:1765-1772; Furlan et al. 1998, Blood 91:2839-2846). Thus, differential diagnosis of TTP can be made by measuring ADAMTS 13 activity.

There are two forms of TTP - congenital (familial) and acquired (Furlan et al. 1996, Blood 87:4223-4234; Furlan et al. 1998, Blood 91:2839-2846). Congenital TTP is caused by genetic mutations in the ADAMTS 13 gene, which result in a loss in ADAMTS 13 production and/or the production of a non-functional ADAMTS 13 enzyme. Acquired TTP is an autoimmune-like disease, which has been linked to intake of certain pharmaceutical drugs. Acquired TTP is caused by the generation of autoantibodies to ADAMTS 13 protein. The onset of acquired TTP has been linked to intake of certain pharmaceutical drugs.

Several methods for measuring the presence and/or activity of ADAMTS 13 are known in the art. These methods include collagen binding assays, ristocetin cofactor assays, electrophoretic analysis (*e.g*. multimer analysis) and immunological methods. Electrophoretic immunoassays, such as Western blotting analysis, have largely been replaced by immunoradiometric assays (IRMA) and enzyme-linked immunosorbant assays (ELISA) (Laffan et al. 2004, Haemophilia 10:199-217). Several reports describe ADAPTS13 assays where the A2 domain of VWF is used as a substrate (Zhou et al. 2004, Thromb. Haemost. 91:806-811; Kokame et al. 2004, Hemost. Thromb. Vasc. Biol. Blood 103:607-612; Cruz et al. 2004, Thromb. Haemost. 90:1204-1209). Cleavage of the A2 domain is monitored by an ELISA method. The available assays for ADAMTS 13 require a relatively high technical skill level, and are therefore not performed in most clinical laboratories. In addition, obtaining results can take several days using available tests, which is detrimental to patients presenting with TTP, who require rapid diagnosis.

WO 2004/035778 pertains to a method for measuring plasma ADAMTS13.

WO 02/10437 describes a method of detecting VWF-cleaning protease using monomeric VWF.

A simple, specific and rapid assay for ADMTS 13 is needed to solve the problems with the currently available assays; yet one has not been developed to date. Attempts at developing such an assay using ADAMTS 13 isolated from plasma have not been successful. Furlan *et al.* tested 28 synthetic chromogenic peptidyl substrates with para-nitroaniline (pNA) as the leaving group, and did not observe consistent, repeatable results (2002 Seminars in Thrombosis and Hemostasis 28(2):167-172). These results demonstrate the difficulty in the art of developing a rapid, reliable assay for ADAMTS 13 activity.

We have discovered that ADAMTS13 on platelets has an enhanced ability, relative to ADAMTS 13 in plasma, to cleave small peptidyl substrates. The difference between plasma and platelet ADAMTS13 is likely due to the finding that platelet ADAMTS13 is cleaved, while plasma ADAMTS13 is not cleaved and remains a single polypeptide. The ADAPTS13 activity on platelets can be enhanced by treatment with coagulation Factor XIa (FXIa). This indicates that activated FXI (FXIa) may cause cleavage of ADAMTS13.We have developed diagnostic chromogenic assays for measuring ADAMTS 13 activity on platelets and in plasma using different peptidyl substrates with leaving groups other than pNA. We have also developed an assay method to measure autoantibodies to ADAMTS13 and an ELISA for measuring ADAMTS13 protein in plasma.

### SUMMARY OF THE INVENTION

In one aspect, the invention provides a method for measuring ADAMTS 13 activity on the surface of platelets according to claim 1.

In a further aspect, the invention provides a method for identifying an inhibitor of ADAMTS13 on the surface of platelets according to claim 7.

In another aspect the invention provides a use of the method for measuring ADAMTS13 activity of the invention for diagnosing TTP in a subject according to claim 15.

In yet another aspect, the invention provides a use of the method for measuring ADAMTS13 activity of the invention for diagnosing acquired TTP in a subject according to claim 23.

The invention additionally provides a use of the method for measuring ADAMTS13 activity of the invention for monitoring treatement of a patient with TTP according to claim 29.

### BRIEF DESCRIPTION OF THE DRAWINGS

The present invention will now be described by way of example, in which reference will be made to the following Figures in which:
Figure 1 shows the structure of the plasma form of ADAMTS13. S: signal peptide; P: propeptide; Dis: disintegrin-like region; Cys: Cys-rich region. The catalytic (metalloprotease) region and the two CUB domains are also shown. Thrombospondin type I (TSP1) repeats are shown as numbered circles.
Figure 2 shows LVY-pNA as a substrate for ADAMTS13.
Figures 3 shows a comparison of LVY-AMC and Suc-LLVY-AMC substrates for measuring ADAMTS 13 activity.
Figure 4 shows the measurement of cleavage of an ADAMTS 13 substrate using fluorescence resonance energy transfer (FRET). The substrate, NH₂-Arg-Lys(*DABCYL*)-NLVYMVTGD(*EDANS*)-Arg-COOH, was incubated with normal platelets, and the increase in fluorescence over time was measured.
Figure 5 shows ADAMTS13 activity in tissue culture fluid from mock transfected and ADAMTS13 transfected HEK 293 cells. Activity was measured using Suc-LLVY-AMC fluorescent substrate.
Figure 6 shows a comparison of ADAMTS 13 activity in plasma from normal subjects and from subjects with acquired TTP using Suc-LLVY-AMC fluorescent substrate.
Figure 7 shows the quantitation of ADAMTS 13 activity in PRP from normal subjects (■) and subjects with acquired TTP (◆).
Figure 8 shows the measurement of ADAMTS13 activity in platelet rich plasma (PRP) and platelet poor plasma (PPP) from six individual samples taken from normal subjects. ADAMTS 13 activity was measured using Suc-LLVY-AMC fluorescent substrate.
Figure 9 shows a comparison of ADAMTS 13 activity per mg of protein on platelets from normal subjects and subjects with acquired TTP.

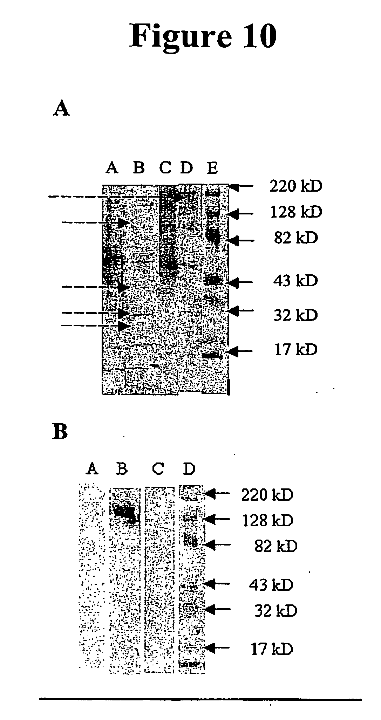

Figure 10 shows immunostaining Western blots of platelet proteins by various anti-ADAMTS 13 antibodies. Figure 10A shows platelet proteins electrophoresed using reducing conditions (lanes A and B) and non-reducing conditions (lanes C and D). Lanes A and C were stained with goat anti-PAI-1 antibody as a control; Lanes B and D were stained with goat anti-ADAMTS 13 antibody; Lane E contains molecular weight standards. Arrows on the left indicate protein bands specifically immunostained by anti-ADAMTS 13 antibodies. Figure 10B shows immunostaining of platelet proteins using human antibodies. Lane A: reducing conditions and Ig from a subject with acquired TTP; Lane B: non-reducing conditions and Ig from a subject with acquired TTP; Lane C: non-reducing conditions and Ig from a normal subject (control); Lane D: molecular weight markers.
Figure 11 shows that ADAMTS 13 activity on normal platelets and on platelets from a subject with acquired TTP is enhanced by treatment with the peptidase activated Factor XIa (FXIa).
Figure 12 shows a plot of FXIa activated platelet ADAMTS 13 activity in normal plasma, and its inhibition by plasma from a subject with acquired TTP.
Figure 13 shows a standard curve depicting concentration dependent increase in optical density using the ADAMTS 13 ELISA and different amounts of normal plasma.
Figure 14 shows the determination of ADAMTS 13 levels, relative to pooled normal plasma (PNP), in twenty-four individual plasma samples from normal subjects using the ELISA method.
Figure 15 shows inhibition of recombinant ADAMTS 13 activity by goat anti-ADAMTS 13 antibody.
Figure 16 shows recombinant ADAMTS 13 activity in the presence of varying amounts of normal plasma or plasma from subjects with acquired TTP, which contains an inhibitor of the enzyme.
Figure 17 shows inhibition of ADAMTS 13 activity on isolated platelets by anti-ADAMTS 13 antibodies from different species.
Figure 18 shows the results of an experiment measuring anti-ADAMTS 13 autoantibodies in plasma by preincubating sample plasma with isolated normal platelets.
Figure 19 shows concentration dependent inhibition of platelet ADAMTS 13 activity by human ADAMTS 13 antibodies in plasma from subjects with acquired TTP.
Figure 20 shows the effect of using different amounts of platelets in the fluorescence assay of the invention. ADAMTS 13 activity was measured in PRP from normal subjects and subjects with acquired TTP.

### DETAILED DESCRIPTION

In this disclosure, "comprises", "comprising", "containing", "having" and the like can have the meaning ascribed to them in U.S. Patent law and can mean " includes", "including", and the like. "Consisting essentially of" or "consists essentially of" likewise have the meaning ascribed in U.S. Patent law and the term is open-ended, allowing for the presence of more than that which is recited so long as basic or novel characteristics of that which is recited is not changed by the presence of more than that which is recited, but excludes prior art embodiments.

### ADAMTS13

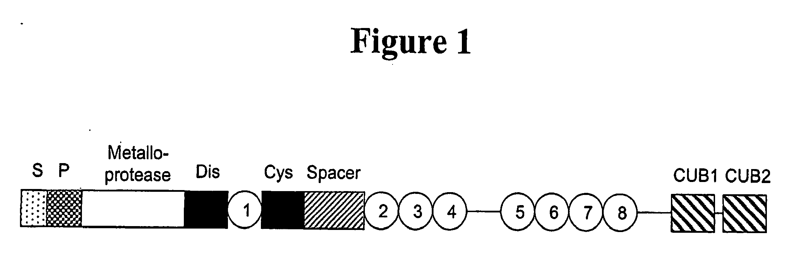

ADAMTS 13, also known as von Willebrand factor (VWF)-cleaving protease, is a member of the family of metalloproteases named for the characteristic combination of a disintegrin-like and metalloprotease (reprolysin-type), with thrombospondin type 1 motifs. The structure of plasma ADAMTS13 is shown in Figure 1. Structural details and sequence information on ADAMTS13 can be found in Zheng et al. (2001; J. Biol. Chem. 276(44):41059-41063). ADAMTS13 cleaves VWF at the Tyr¹⁶⁰⁵-Met¹⁶⁰⁶ bond and requires both calcium and zinc ions to function.

Until the instant invention, ADAMTS 13 activity was primarily studied in plasma. This form of ADAMTS 13 is referred to herein as "plasma ADAMTS13". A novel form of ADAMTS 13 has now been discovered on platelets and is referred to herein as "platelet ADAMTS 13". "Recombinant ADAMTS13" can also be made using standard molecular biology techniques, such as those found in Sambrook, et al., Molecular Cloning, A Laboratory Manual (1989) and Ausubel et al., Short Protocols in Molecular Biology (1999) 4th Ed., John Wiley & Sons, Inc. (as well as the complete version of Current Protocols in Molecular Biology).

Therefore, described herein is an ADAMTS13 protein and ADAMTS13 activity in platelets. This protein appears to be distinct from plasma ADAMTS 13 in that plasma ADAMTS 13 is one contiguous polypeptide, whereas platelet ADAMTS 13 protein is cleaved into more than one polypeptide, which is then held together by disulfide bonds. Polypeptides of about 120 kD, about 84 kD, about 60 kD, about 43 kD and about 30 kD are seen on an SDS-PAGE gel of platelet ADAMTS13. Platelet ADAMTS13 appears to be cleaved by FXIa. Likewise, this form of ADAMTS13 also appears to be distinct from that reported by Suzuki et al. (2004, Biochem. Biophys, Res. Commun. 313:212-216). Unlike the cleaved platelet ADAMTS13 of the instant invention, the ADAMTS 13 of Suzuki *et al.* has a molecular weight of about 220 kD on an SDS-PAGE gel. Moreover, Suzuki *et al.* report the larger form of ADAMTS 13 in platelets, while experiments performed by the current inventors indicate ADAMTS 13 activity on the surface of platelets. Without wishing to be bound by theory, it is possible that the platelet ADAMTS13 of the invention has undergone different post-translational modification than that of Suzuki *et al.,* resulting in a cleaved surface protein, rather than a larger cytoplasmic protein.

The assays of the invention can be used to detect ADAMTS13 on the surface of platelets.

The source of ADAMTS13 for use in the assays and methods of the invention are isolated platelets. Methods for making platelet rich plasma (PRP), platelet poor plasma (PPP), isolated platelets and tissue culture supernatant can be found in the Examples section. Purified ADAMTS13 can be made from plasma, platelets or recombinant cells using standard biochemical techniques for protein isolation and purification including, but not limited to, immunoaffinity chromatography, size exclusion chromatography, ion exchange chromatography, immunoprecipitation, and ammonium sulfate precipitation. The term "plasma" can include PRP, PPP and pooled normal plasma (PNP).

As used herein, "normal platelets", "normal plasma", "normal PRP", *etc.* are derived from individuals who do not have either congenital TTP or acquired TTP. PNP is a mixture of plasma taken from multiple individuals who do not have TTP. Likewise, "TTP platelets", "TTP plasma", "TTP PRP", *etc.* are derived from individuals who have either congenital TTP or acquired TTP. "Acquired TTP platelets", "acquired TTP plasma", "acquired TTP PRP", *etc.* are derived from individuals who have the acquired form of TTP.

Measurements of ADAMTS 13 activity are made in relation to "normal activity", that is, ADAMTS 13 activity in normal platelets, normal plasma, normal PRP, recombinant or purified ADAMTS 13 *etc.* Thus, terms such as "reduced ADAMTS 13 activity" or "inhibited ADAMTS 13 activity" refer to ADAMTS 13 activity relative to activity measured in a normal sample, *i.e*. normal platelets, normal plasma, normal PRP, recombinant or purified ADAMTS13 *etc*.

The methods of the invention are applicable to clinical, veterinary and/or research applications.

### Substrates

The invention relates to assays for measuring ADAMTS13 on the surface of platelets activity. In one such assay, a sample comprising ADAMTS 13 is incubated with a substrate that comprises a peptide moiety and a chromogenic moiety and the optical density or fluorescence of the sample is measured, thereby measuring ADAMTS 13 activity. In this embodiment, the peptide moiety comprises X-Val-Tyr, X-Leu-Tyr or X-Ile-Tyr, wherein X is any amino acid. In a preferred embodiment, X is Leu. In other preferred embodiments, the peptide moiety is Leu-Val-Tyr, Leu-Leu-Val-Tyr or Suc-Leu-Leu-Val-Tyr.

The skilled artisan can make amino acid substitutions in the peptide moiety without undue experimentation. For example, amino acid substitutions may be made on the basis of similarity in polarity, charge, solubility, hydrophobicity, hydrophilicity, and/or the amphipathic nature of the residues as long as the binding activity of the substrate is retained. For example, negatively charged amino acids include aspartic acid and glutamic acid; positively charged amino acids include lysine and arginine; and amino acids with uncharged polar head groups having similar hydrophilicity values include leucine, isoleucine, valine, glycine, alanine, asparagine, glutamine, serine, threonine, phenylalanine, and tyrosine.

Conservative substitutions may be made, for example, according to the Table below. Amino acids in the same block in the second column and preferably in the same line in the third column may be substituted for each other:

| | | |
|---|---|---|
| ALIPHATIC | Non-polar | G A P |
| | | I L V |
| | Polar - uncharged | C S T M |
| | | N Q |
| | Polar-charged | D E |
| | | K R |
| AROMATIC | | H F W Y |

The peptide moiety is in the range of about 3 to about 20 amino acid residues in length. Non-conventional amino acids such as those found in the Spectrozyme series of peptidyl substrates from American Diagnostica.Inc. (Stamford, CT) may also be used to substitute for conventional amino acids, provided that the peptide retains its ability to bind to and be cleaved by ADAMTS 13.

Chromogenic moieties suitable for use in the invention include s-benzyl, 5-amino-2-nitrobenzoic acid and 6-amino-1-naphthalenesulfonamides. The chromogenic moiety is not para-nitroanaline (pNA), which has been demonstrated to be an ineffective moiety for an ADAMTS13 substrate. Without wishing to be bound by theory, it is possible that the structure of pNA creates a steric hindrance that prevents ADAMTS 13 activity.

Another assay described herein a method for measuring ADAMTS13 activity by incubating a sample comprising ADAMTS 13 with a substrate having donor and acceptor moieties that mediate fluorescence resonance energy transfer (FRET). In addition to the donor and acceptor moieties, the substrate comprises a peptide moiety that comprises Val-Tyr-Met, Leu-Tyr-Met or Ile-Tyr-Met. ADAMTS 13 activity is determined by measuring the fluorescence of the sample.

FRET is a distance-dependent interaction between the electronic excited states of two dye molecules in which excitation is transferred from a donor moiety to an acceptor moiety without emission of a photon. This is accomplished by using donor/acceptor pairs in which the emission spectrum of the donor overlaps the absorption spectrum of the acceptor. When the two are in spatial proximity, the excitation energy of the donor is transferred to the acceptor through long-range dipole-dipole interactions. When energy transfer occurs, the acceptor quenches the fluorescence of the donor, and thus, the acceptor moiety is also called a quencher. The donor and acceptor moieties must be within about 100 angstroms or 10 nm of one another for efficient energy transfer.

Suitable donor/acceptor pairs for use in FRET are well known in the art. Examples of donor/acceptor pairs can be found in Table 1. It should be noted that Table 1 does not contain an exhaustive list of FRET donor/acceptor pairs, and that the skilled artisan can choose a donor/acceptor pair based on his or her knowledge of the art.

**Table 1. FRET Donor and Acceptor Moieties**

| **Donor** | **Acceptor** |
|---|---|
| 7-Methoxycoumarin | DABCYL |
| Amino Methyl Coumarin | DABCYL, QSY -35¹ |
| Blue Fluorescent Protein | Ds Red Fluorescent Protein |
| BODIPY-FL² | DABCYL, QSY-7, QSY-9, BHQ-1³ |
| B-Phycoerythrin | Cy5 |
| Carboxyfluorescein Succinimidyl Ester | Texas Red |
| Cascade Blue | DABCYL |
| Coumarin | DABCYL |
| Cy3 | Cy5, QSY-7, QSY-9, BHQ-2 |
| Cy5 | Cy5.5, QSY-35, BHQ-2 |
| Dansyl | FITC |
| Dansyl | Octadecylrhodamine |
| Dialkylaminocoumarin | DABCYL, QSY-35 |
| EDANS⁴ | DABCYL |
| FITC | Eosin Thiosemicarbazide |
| Fluorescein | Tetramethylrhodamine |
| Green Fluorescent Protein | Yellow Fluorescent Protein |
| IAEDANS⁵ | DDPM⁶ |
| Marina Blue | DABCYL, QSY-35 |
| Pacific Blue | DABCYL, QSY-35 |
| Rhodamine 6G | Malachite Green |
| Tryptophan | Dansyl |

| | |
|---|---|
| ¹ QSY quenchers are analogs of fluorescein. ² 4,4-difluoro-4-bora-3a,4a-diaza-s-indacene ³ Black Hole Quenchers^{™} by Biosearch Technologies, Inc., Novato, CA ⁴ 5-[(2-aminoethyl)amino]naphthalene-1-sulfonic acid ⁵ 5-(2-iodoacetylaminoethyl)aminonaphthalene-1-sulfonic acid ⁶ N-(4-dimethylamino-3,5-dinitrophenyl)maleimide | |

The preferred donor/acceptor pair is EDANS/DABCYL.

Preferably, the peptide moiety is at least about 3 amino acid residues in length. More preferably, the peptide moiety is in the range of about 3 to about 30 amino acid residues in length. Advantageously, the peptide moiety is Asn-Leu-Val-Tyr-Met-Val-Thr-Gly-Asp. Amino acid substitutions can be made as described above without departing from the spirit and scope of the invention.

The preferred substrate for use in this embodiment of the invention is NH₂-Arg-Lys-(*DABCYL*)*-* Asn-Leu- Val-Tyr-Met- Val- Thr-Gly-Asp-(*EDANS*)-Arg-COOH.

Methods for making the ADAMTS13 substrates are also disclosed. Said methods comprise covalently linking a peptide moiety, as described above, to a chromogenic or fluorogenic moiety, or to a donor moiety and an acceptor moiety that mediate FRET, using well known synthesis techniques.

### Inhibitors of ADAMTS13

Described herein is a method for inhibiting ADAMTS13 using an inhibitory antibody against ADAMTS13. Several anti-ADAMTS13 antibodies are available from various sources. For instance, Examples 12 and 14 demonstrate the inhibitory effects of anti-ADAMTS 13 antibodies from goat, rabbit and human.

Autoantibodies that inhibit ADAMTS 13 activity can be detected in subjects with acquired TTP. (See Klaus et al. 2004, Blood 103(12):4514-4519.) These antibodies can be isolated from the plasma of acquired TTP patients and used *in vitro* as inhibitors of ADAMTS 13. Moreover, a method of diagnosing acquired TTP provided by the instant invention is to incubate an ADAMTS13 sample with plasma from a subject to be tested for acquired TTP and to measure ADAMTS13 activity on the surface of platelets using one of the assays of the invention. Inhibition of ADAMTS 13 activity by the plasma indicates the presence of inhibitory anti-ADAMTS13 antibodies in the plasma, and thus confirms a diagnosis of acquired TTP.

Klaus *et al.* (*Ibid*) performed epitope mapping experiments in order to deduce the nature of inhibitory ADAMTS 13 antibodies in patients with acquired TTP. The results indicate that antibodies directed against the cysteine rich/spacer domain of ADAMTS13 were detected in all cases. In addition, antibodies directed against the TSP1-1 repeat or a fragment containing the TSP1-1 repeat, the disintegrin-like domain and the catalytic domain were detected in 72% of patients. Further, antibodies directed against the CUB 1 and/or CUB 2 domains were detected in 64% of patients. For purposes of this invention, the C-terminus consists of thrombospondin type 1 TSP1 repeats 2-8 and the CUB domains. Figure 1 shows a schematic diagram of ADAMTS13.

The invention provides a method for identifying inhibitors of ADAMTS13 on the surface of platelets. The inhibitor can be, *inter alia,* a polypeptide, a peptide, an oligonucleotide, a polynucleotide, a nucleoside or nucleoside analog, a saccharide, a small molecule, or a natural or synthetic chemical. The method comprises incubating a sample of ADAMTS13 with a candidate inhibitor and measuring ADAMTS13 activity on the surface of platelets according to one or more of the assays provided by the invention. The inhibitor is identified by reduced ADAMTS13 activity on the surface of platelets in the test sample, compared with ADAMTS13 activity on the surface of platelets in a control sample not incubated with the candidate inhibitor. The candidate inhibitor can be generated using known techniques, and can be derived, for example, from a chemical compound library, a phage display library, a natural chemical library or a combinatorial chemistry library.

### ELISA

The Enzyme Linked Immunosorbent Assay (ELISA) is a solid-phase immunoassay that is widely used in both clinical and basic research settings. In one type of ELISA, an antigen is attached to the solid phase, which is most commonly a membrane, plate, microwell or bead. The solid phase is then incubated with an antibody to the antigen (a "primary antibody"). If the primary antibody is conjugated to a label, it can be detected. This process is known as direct immunolabelling. Alternatively, the primary antibody may be unlabelled, in which case an antibody to the primary antibody (a "secondary antibody"), raised in a different species from the primary antibody and conjugated to a label, is incubated with the solid phase. This detection method is referred to as indirect immunolabelling. Immunolabelling methods are well known in the art.

In a different type of ELISA, an antibody is attached to the solid phase. This antibody can then be used to capture an antigen of interest. Direct or indirect immunolabelling techniques can be employed for the sake of analysis. ADAMTS 13 in a sample can be measured by binding an anti-ADAMTS13 antibody to a solid phase and adding the sample to the solid phase. ADAMTS 13 present in the sample binds to the antibody, and bound ADAMTS 13 is detected using direct or indirect immunolabelling. The amount of ADAMTS 13 in the sample can be measured by quantifying the label.

The sample is or comprises platelets or is plasma.

Another ELISA disclosed herein is used to detect anti-ADAMTS13 antibodies in a test sample. In this embodiment, an anti-ADAMTS 13 antibody raised in a first species, *e.g*. a goat, is bound to a solid phase. ADAMTS 13 is added and binds to the antibody raised in the first species. A test sample from a second species, *e.g*. human, is added, and any anti-ADAMTS13 antibodies present in the test sample bind to the ADAMTS13. Finally, a labelled antibody against antibodies from the second species is added. The labelled antibody is raised in a third species, *e.g*. donkey, and binds to the antibody from the second species. By detecting the label, one can detect anti-ADAMTS13 antibodies in the test sample.

The test sample can be any biological fluid, such as blood, plasma, serum, culture fluid, cerebralspinal fluid or sputum.

The label can be any moiety known in the art, including chromogenic enzyme systems, such as horseradish peroxidase, alkaline phosphatase, β-galactosidase, glucose 6-phosphate dehydrogenase. These enzyme labels are reacted with a chromogenic substrate that can be detected by conventional techniques. The label could be horse radish peroxidase, and the substrate is tetramethylbenzidine. The label can also be a fluorescent dye, including rhodamine, fluorescein, Cy dyes, Texas Red, and derivatives thereof.

### Diagnostic Applications

In addition to the method discussed above for diagnosing acquired TTP by testing the inhibitory properties of plasma on ADAMTS13 on the surface of platelets the invention provides other methods for the diagnosis of both congenital and acquired TTP.

For example, congenital or acquired TTP can be diagnosed by measuring ADAMTS13 activity on the surface of platelets in a test sample from a subject using one of the assays of the invention and subsequently comparing the ADAMTS 13 activity on the surface of platelets in the test sample to ADAMTS13 activity on the surface of platelets in a control sample having normal ADAMTS13 activity on the surface of platelets. Reduced ADAMTS13 activity on the surface of platelets in the test sample compared with the control sample indicates a positive diagnosis of TTP.

Acquired TTP can be diagnosed in a subject by incubating a sample comprising ADAMTS 13 with plasma from the subject; and measuring ADAPTS13 activity on the surface of platelets in the sample using one of the assays of the invention. Reduced ADAMTS13 activity on the surface of platelets in the sample, compared with ADAMTS13 activity on the surface of platelets in a control sample having normal ADAMTS13 activity on the surface of platelets, indicates acquired TTP.

### Treatment Applications

The techniques provided by the invention can be used to monitor treatment of a patient for TTP. For example, during treatment, ADAMTS13 activity on the surface of platelets can be measured using the assays of the invention. This allows the clinician to assess the efficacy of the treatment and/or to determine the length of a treatment session that is required to restore ADAMTS13 activity.

One treatment for TTP is plasmaphoresis. In patients with acquired TTP, plasmaphoresis is used to remove the inhibitory anti-ADAMTS 13 antibodies from the patient's plasma. Replacement of the deficient ADAMTS 13 is provided by infused plasma. Recent advances in our understanding of the pathological mechanisms of TTP provide a rationale for monitoring plasmaphoreseis via measuring the levels of ADAMTS13 activity in the patient undergoing plasmaphoresis.

Because the platelet form of ADAMTS 13 reacts with low molecular weight substrates more efficiently than the plasma form, platelet ADAMTS13 is likely to be a better target for drugs designed to treat TTP. Therefore, disclosed herein is a method for treating TTP in a patient in need thereof comprising administering to the patient a drug that specifically targets platelet ADAMTS13. The drug can be identified using the screening methods provided by the invention and described herein. The term "drug" is meant to encompass a polypeptide, a peptide, an oligonucleotide, a polynucleotide or vector containing a polynucleotide, a nucleoside or nucleoside analog, a saccharide, a small molecule, or a natural or synthetic chemical.

The invention will now be further described by way of the following non-limiting Examples, given by way of illustration.

### EXAMPLES

### Materials and Methods

### Synthetic Peptidyl Substrates

Leu-Val-Tyr-AMC (LVY-AMC) and Suc-Leu-Leu-Val-Tyr-AMC (Suc-LLVY-AMC) were obtained from Bachem Bioscience Inc. (King of Prussia, PA). LVY-AMC was also made by QPR (Montreal, Canada). FRET substrate, NH₂-Arg-Lys(*DABCYL*)-NLVYMVTGD(*EDANS*)-Arg-COOH, was made for American Diagnostica Inc. by Molecular Biology Resource Center, University of Oklahoma Health Sciences Center (Oklahoma City, OK).

### Preparation of Platelet Rich Plasma (PRP), Platelet Poor Plasma (PPP) and Isolated Platelets

Blood was collected by venopuncture into collection tubes containing 0.12 M sodium citrate, an anti-coagulant. The citrated blood was centrifuged at 700 rpm for 10 minutes. The supernatant was removed and saved as the platelet rich plasma (PRP) fraction. The PRP was centrifuged at 10,000 rpm for 20 minutes. The supernatant was removed and saved as the platelet poor plasma (PPP) fraction. The pellet contained the platelets, which were washed by resuspending in 10 mM Tris-HCl pH 8.0 buffer and centrifuging at 10,000 rpm for 10 minutes. The isolated platelets were resuspended in buffer and used in experiments.

### Recombinant ADAMTS 13

HEK 293 cells were transfected with a vector encoding ADAMTS13. Cell culture supernatant from the transfected HEK 293 cells, containing recombinant ADAMTS13, was generously provided by Dr. David Ginsburg (University of Michigan, MI). Mock transfected cell supernatant with no recombinant ADAMTS 13 was also provided as a control.

### Example 1. Measurement of ADAMTS13 Activity in PRP Using LVY-pNA and LVY-AMC Peptidyl Substrates

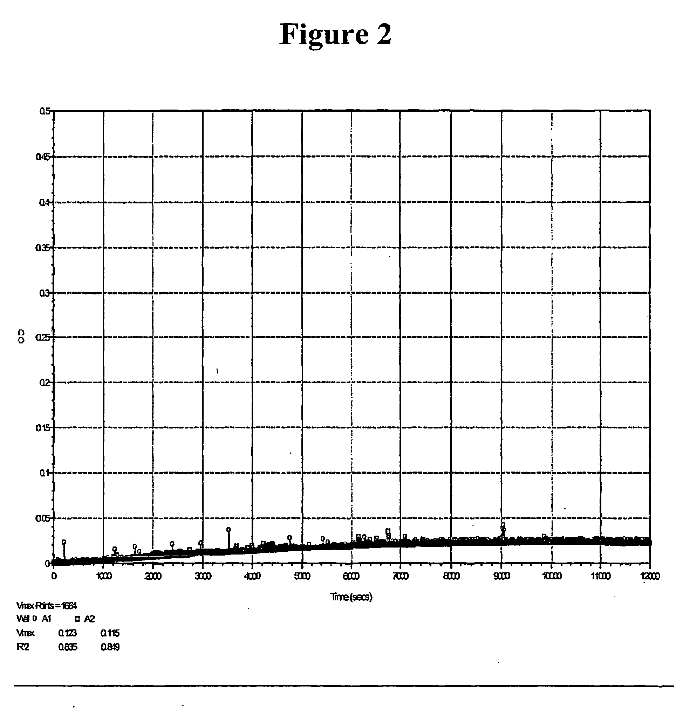

As discussed above, Furlan *et al.* reported that some peptidyl-pNA moieties, such as XLY-pNA and XVY-pNA, were not substrates for ADAMTS13. We prepared LVY-pNA and tested it for its ability to be cleaved by ADAMTS 13. The results shown in Figure 2 confirm the findings of Furlan *et al., i.e.,* LVY-pNA was not cleaved by ADAMTS13.

Thus, the nature of the leaving group of the peptidyl substrate appears to be important in determining the ability of ADAMTS 13 to cleave a chromophor or a fluorophor from the end of a peptidyl substrate.

### Example 2. Measurement of ADAMTS13 Activity in PRP Using Peptidyl Substrates of Different Lengths

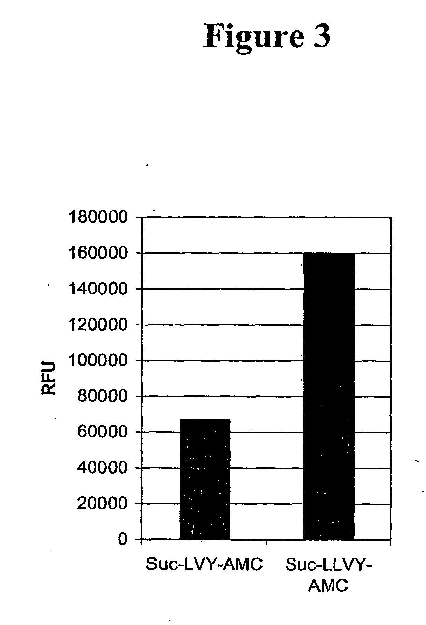

In order to determine the effect of modifying the length of the peptide sequence conjugated to the AMC fluorochrome, normal PRP (20 µl) was incubated at 37° C with a final concentration of 0.8 mM Suc-LLVY-AMC or LVY-AMC in 10 mM Tris-HCl pH 8.0 buffer in total volume of 200 µl. Fluorescence was measured using a microtiter fluorophotometric plate reader (Ex 360 nm/ Em 460 nm). Both peptidyl-AMC substrates were cleaved by ADAMTS13 in PRP, with the Suc-LLVY-AMC giving a higher signal over time as compared to the LVY-AMC substrate (Figure 3).

This example demonstrates that different ADAMTS 13 substrates can be made by altering the peptide sequence that is conjugated to the AMC fluorophor.

### Example 3. Measurement of ADAMTS13 Activity Using a FRET Substrate

ADAMTS13 activity was measured using a fluorescent substrate attached to a donor moiety and an acceptor moiety that functions by fluorescence resonance energy transfer (FRET). The ADAMTS 13 selective substrate, NH₂-Arg-Lys(*DABCYL*)-NLVYMVTGD(*EDANS*)-Arg-COOH, was used in this example. The NLVYMVTGD peptide sequence of this substrate is homologous to the ADAMTS13 cleavage site on VWF. The intact peptidyl substrate has low fluorescence, due to quenching of the DABCYL-fluorochrome by the EDANS-quencher. Cleavage of the substrate between the tyrosine and methionine of the peptide sequence results in an increase in fluorescence.

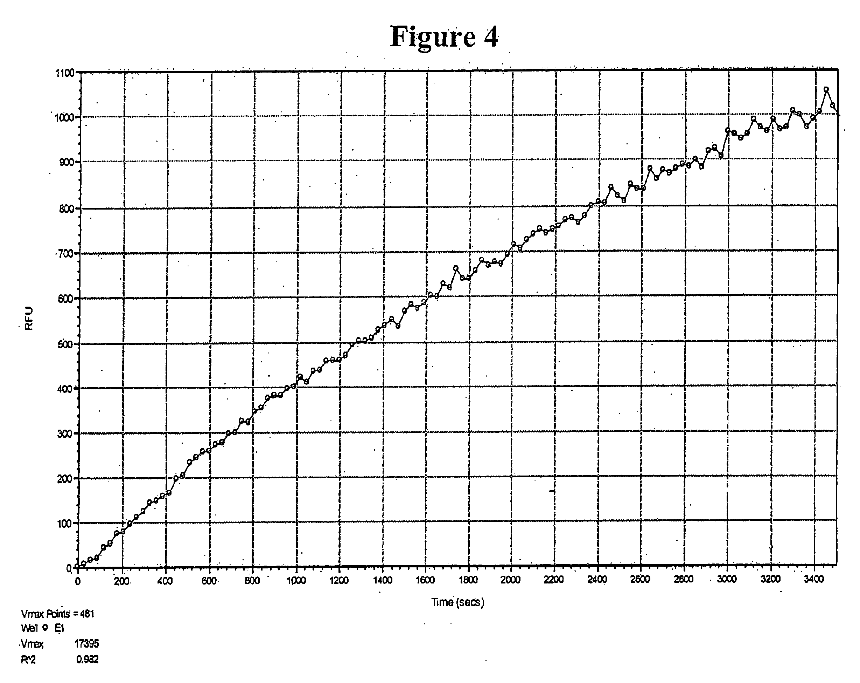

Isolated platelets in 10mM Tris-HCl pH 8.0 assay buffer were incubated with a final concentration of 8 µg/ml of the FRET substrate at 37°C. The fluorescence was measured in a spectrofluorometric plate reader (Ex 360 nm/ Em 440 nm). Figure 4 shows that incubation of platelets with NH₂-Arg-Lys(*DABCYL*)-NLVYMVTGD(*EDANS*)-Arg-COOH results in increased fluorescence over time, indicating that the form of ADAMTS13 found on platelets cleaves this FRET peptide substrate. The skilled artisan can design other FRET substrates for measuring ADAMTS 13 activity using his or her own knowledge and the teachings herein.

### Example 4. Measurement of Recombinant ADAMTS13 Activity in Tissue Culture Supernatants Using Suc-LLVY-AMC Fluorescent Substrate

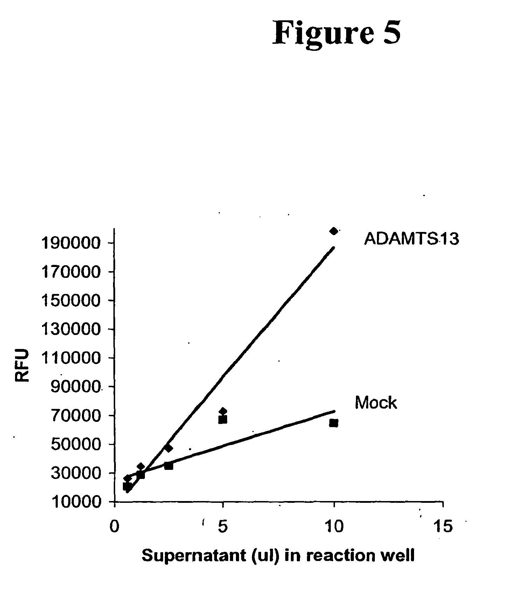

Fluorescence signals were compared between tissue culture supernatant from HEK 293 cells transfected with a viral vector coding for recombinant ADAMTS 13 ("recADAMTS13") and supernatant from mock transfected HEK 293 cells, as a control. (Culture supernatants were supplied by Dr. David Ginsburg, University of Michigan.) Varying amounts of the two culture supernatants were added to 0.8 mM Suc-Leu-Leu-Val-Tyr-AMC fluorescent substrate in 50 mM Tris-HCl pH 8.0 in a total volume of 100 µl. Figure 5 shows that a greater fluorescence signal was produced by the supernatant from transfected cells verses supernatant from mock transfected cells. The increased fluorescence signal is due to cleavage of the substrate by recADAMTS13 in supernatant of the vector transfected cell supernatant.

### Example 5. Measurement of ADAMTS13 Activity in Plasma

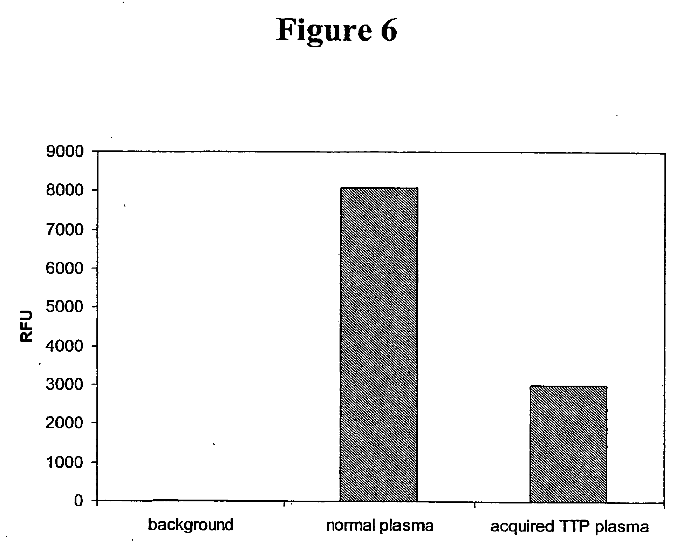

ADAMTS 13 activity was measured in plasma using the Suc-LLVY-AMC substrate. Normal plasma (50 µl) or acquired TTP plasma (50 µl) was added to substrate in 50 mM Tris-HCl pH 8.0 buffer. The final concentration of the substrate was 0.8 mM; substrate in buffer was used as a background control. The reaction mixture was incubated at 37° C for 4 hours and fluorescence was recorded in a spectrofluorometric plate reader at Ex 360 nM/ Em 440 nM (Figure 6). Higher ADAMTS 13 activity was observed in the normal plasma as compared to acquired TTP plasma.

### Example 6. Comparison of ADAMTS13 Activity in PRP from Normal and TTP Subjects

The ADAMTS 13 activity from normal plasma and from acquired TTP plasma were compared in the fluorescence assay described above. Specifically, 20 µl of normal PRP were serially diluted 1:2 in normal PPP in microtiter wells. Eighty µl of LVY-AMC (0.2 mM final concentration) in 10 mM Tris-HCl pH 8.0 buffer were added to the PRP. The microtiter plate was placed in a spectrofluorometric microtiter plate reader at 37° C and the fluorescence was monitored at Ex 360 nm/ Em 440 nm for 16 minutes.

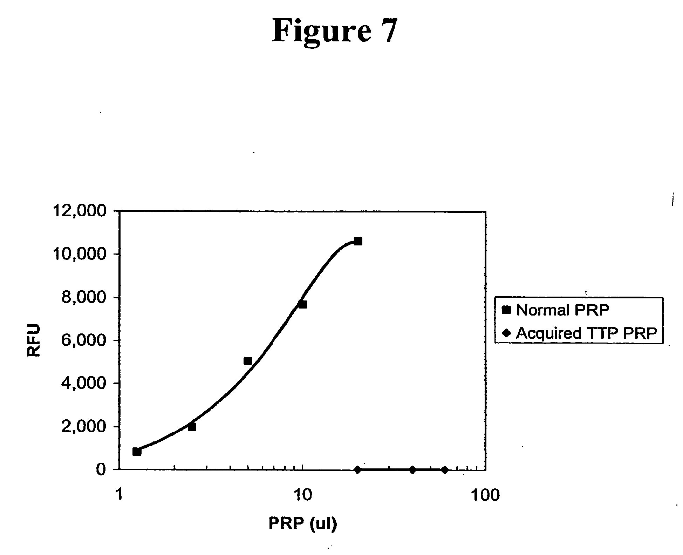

The results shown in Figure 7 demonstrate that, as the amount of PRP in the assay decreased, there was a decrease in the fluorescence signal. PRP from a patient diagnosed with acquired TTP was tested in the assay under the same conditions as normal PRP. In this experiment, more plasma from the acquired TTP patient (20-60 µl) was used in the assay, as compared to normal PRP. No fluorescent signal was generated at the highest amount of PRP from the acquired TTP patient. This shows that ADAMTS13 activity in PRP from an acquired TTP patient has significantly less activity than normal PRP. This method can be used to diagnose deficiency in ADAMTS13 activity.

### Example 7. ADAMTS13 Activity Is Associated with Platelets

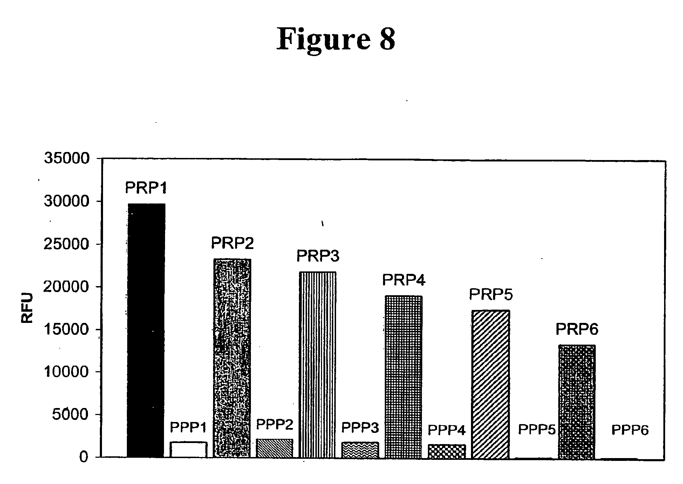

PRP and PPP from six normal volunteers were prepared as described above. Fifty µl of each plasma sample were added to 130 µl 50 mM Tris-HCl buffer pH 8.0 and 20 µl of 8 mM Suc-LLVY-AMC substrate. The reaction mixtures were incubated at 37° C and monitored for 1 hour in a spectrofluormetric plate reader at Ex 360 nm/ Em 440 nm. The six PRP samples exhibited high ADAMTS13 activity, as evidenced by generation of a high fluorescence signal (RFU) over time (Figure 8).

The rate of fluorescence generation over time was significantly greater in PRP than in plasma. After pelleting of platelets, the PPP from each subject was also tested, and exhibited very little ADAMTS 13 activity. These results show that the majority of ADAMTS13 activity in PRP is present on the platelets and not in the plasma.

### Example 8. Reduced ADAMTS13 Activity is Associated with Human Platelets from an Acquired TTP Patient

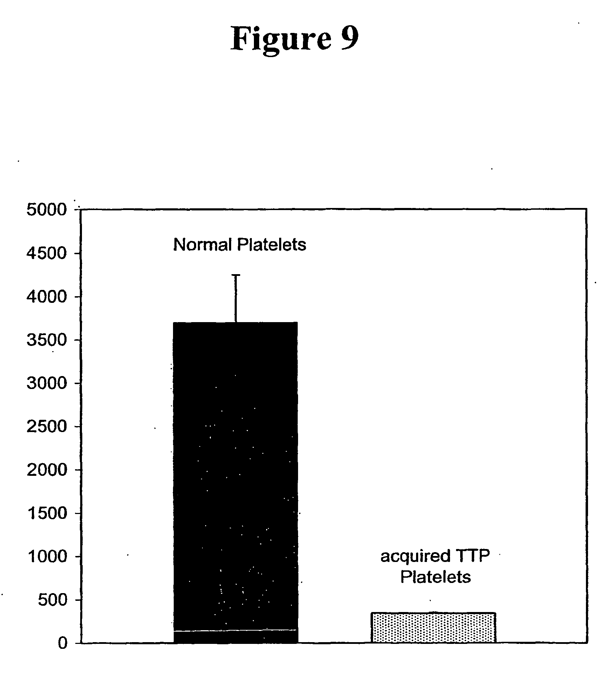

Isolated platelets from a normal subject and from a patient diagnosed with acquired TTP were prepared as described above. Approximately 600 µg of protein in 100 µl of 50 mM Tris-HCl pH 8.0 buffer from the normal and the acquired isolated platelets were placed in wells in a fluorescence microtiter plate. LVY-AMC substrate was added to each tube to a final concentration of 0.2 mM and the reaction mixtures were incubated at 37° C for 1 hour. The fluorescence was monitored at Ex 360 nm/ Em 440 nm. The amount of ADAMTS 13 activity per mg of protein associated with normal platelets was significantly greater than that associated with platelets from a patient with acquired TTP (Figure 9). This method can be used to quantitate ADAMTS13 activity on platelets. Low ADAMTS 13 activity would indicate TTP. Both congenital TTP, caused by mutation or alteration of ADAMTS 13, and acquired TTP, caused by presence of ADAMTS 13 inhibitory antibodies, can be diagnosed using this method.

### Example 9. ADAMTS13 Associated with Platelets is Present in a Cleaved Form

The molecular form of ADAMTS 13 in platelets was investigated by immunostaining of Western blots of platelet proteins (Figure 10). Washed platelets were solubilized in SDS-sample buffer with and without mercaptoethanol. SDS-PAGE electrophoresis was performed using 4-20% acrylamide gel. The resolved proteins were electroblotted onto nylon paper and immunostained with different biotinylated anti-ADAMTS13 antibodies and strepavidin-HRP/TMB. A goat anti-ADAMTS 13 antibody specifically immunostained protein bands under reducing conditions at approximately 120 kD, 43 kD and 30 kD. Using non-reducing conditions, a high molecular weight band at approximately 150 kD was specifically immunostained.

These findings suggest that ADAMTS 13 on platelets is comprised of more than one protein held together by disulfide bonds. ADAMTS 13 in plasma has been reported to be comprised of a single protein of molecular weight 150-170 kD. The ADAMTS13 associated with platelets is different than plasma ADAMTS 13, and appears to be cleaved. ADAMTS 13 from platelets was immunostained using plasma from an acquired TTP patient. A high molecular weight band was specifically immunostained under non-reducing conditions. Reduction of the protein caused the ADAMTS13 not to be stained by this TTP plasma.

### Example 10. Enhanced Activity of Platelet ADAMTS13 by Treatment with a Proteolytic Enzyme

ADAMTS 13 on platelets appears to be proteolytically cleaved into at least two or more polypeptide chains held together by disulfide bonds, whereas plamsa ADAMTS 13 is a single peptide chain. We tested whether a peptidase can cleave ADAMTS13 and increase activity toward ADAMTS 13 peptide substrates.

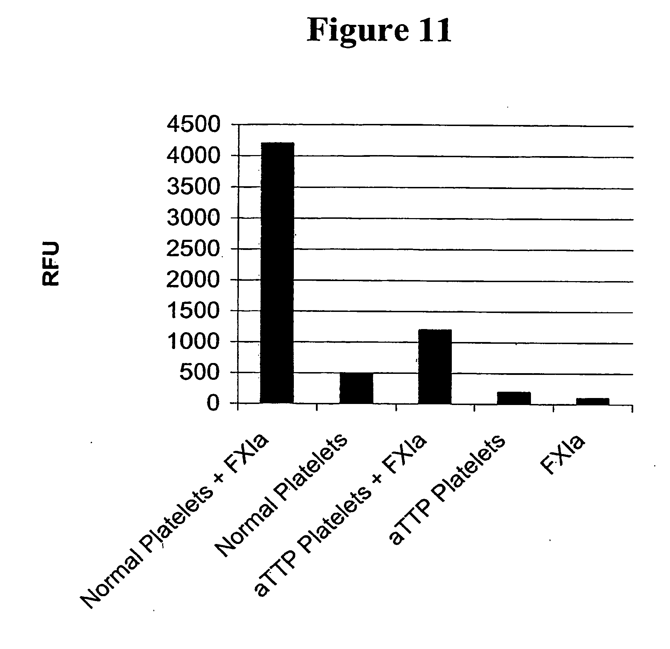

Figure 11 shows that ADAMTS 13 activity on platelets is enhanced by treatment with peptidase activated FXIa. Washed normal platelets from 200 µL of PRP were resuspended in I ml of assay buffer. Washed platelets from 1 ml of PRP from a subject with acquired TTP were resuspended in 50 µL of assay buffer. Normal or TTP platelets (10 µl) were added to 10 mM Tris-HCl pH 8.0 assay buffer (80 µl) or assay buffer containing 0.3 ng/ml of FXIa (80 µl). LVY- AMC substrate (10 µl of 2 mM) was added and the fluorescence was monitored (Ex 360 nm/ Em 440 nm) for 1500 seconds. FXIa at 0.3 ng/ml was used as a control. ADAMTS 13 activity was significantly increased (by approximately 8-fold) towards LVY-AMC substrate upon treatment of platelets with FXIa. Activity of platelets isolated from PRP of an acquired TTP patient was enhanced by treatment with FXIa to a much lesser extent than those isolated from PRP of a normal individual. This shows that the inhibitory antibodies in TTP plasma block activation of platelet ADAMTS 13 by FXIa.

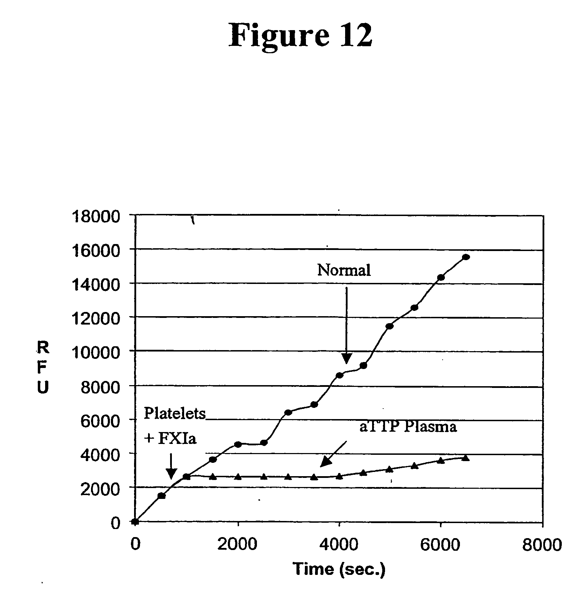

The activity of FXIa treated platelets towards LVY-AMC was significantly reduced after addition at 1500 seconds of 50 µl acquired TTP plasma to the reaction mixture, whereas, addition of 50 µl normal plasma had no significant effect on the activity (Figure 12). These results show that the enhancement of platlelet ADAMTS 13 activity by FXIa is blocked by anti-ADAMTS13 antibodies. These findings also show that ADAMTS13 activity can be enhanced by treatment with proteolytic enzymes.

These findings suggest that ADAMTS 13 present on platelets is in a different form than that found in plasma. Results above show that platelet ADAMTS13 appears to have greater enzymatic activity towards peptidyl substrates than plasma ADAMTS 13. The finding of proteolytically cleaved ADAMTS13 on platelets may explain the differences in reactivity of plasma and platelet-bound ADAMTS 13 towards peptidyl substrates.

### Example 11. Measuring ADAMTS13 Antigen in Plasma Using ELISA

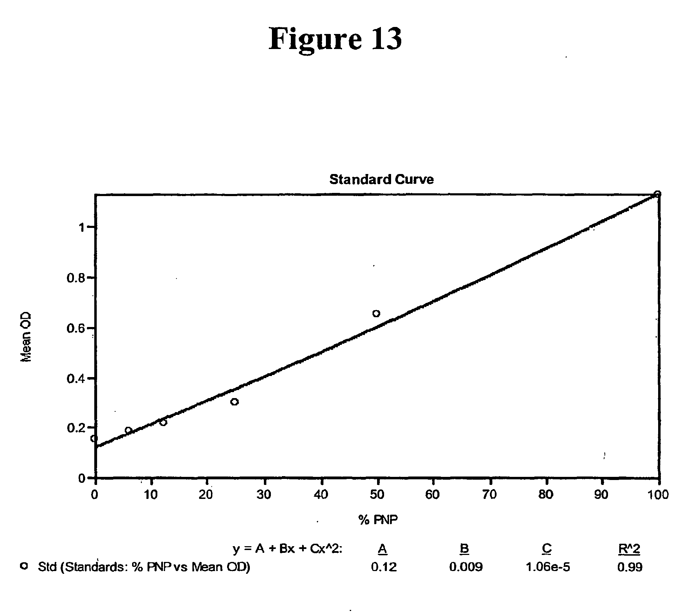

An ELISA for measuring ADAMTS13 protein in biological fluids was developed. Immulon 4 96-well microtiter plates (Dynex) were coated with goat anti-ADAMTS13 antibody (2 µg/ml) in 100 µl of 50.mM MOPS buffer pH 6.0. Plates were washed and blocked with Superblock (Pierce, IN). A PNP sample was serially diluted 1:2 in buffer and 100 µl was added to microtiter wells. After incubation for 1 hour at 37°C, the plate was washed and 0.5 µg/ml immunoglobulin purified from plasma obtained from a patient with acquired TTP was added to the microtiter wells. After incubation at 37° C for 1 hour, the plate was washed and donkey anti-human Ig-HRP labeled antibody (Jackson Laboratories, ME) (1:1000) was added to the wells. After incubation for 1 hour at 37° C, the plate was washed and 100 µl TMB substrate (Moss Inc, MD) was added to each well. The plate was incubated at room temperature for 5 minutes and the reaction was stopped by adding 50 µl of 0.45 M sulfuric acid. The absorbance at 450 nm was measured. Figure 13 shows the increase in absorbance was linearly proportional to the amount of ADAMTS 13-containing plasma added.

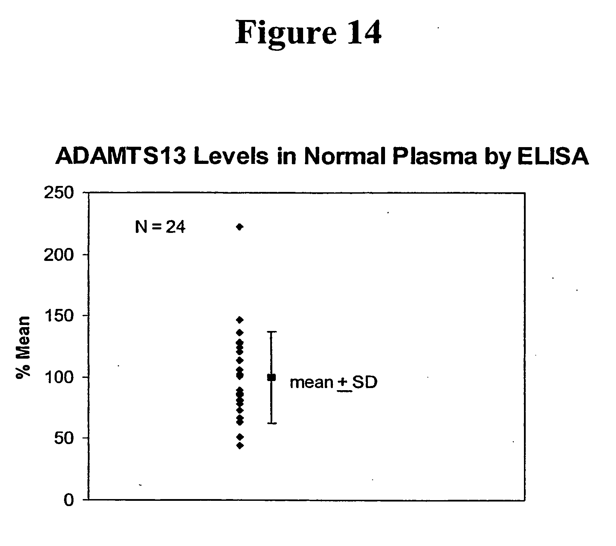

In another experiment, the amount of ADAMTS 13 protein in 24 normal plasma samples was determined using the ELISA method. The results are shown in Figure 14. Using PNP as 100%, the amount of ADAMTS 13 in 24 individual normal plasma samples was distributed around the PNP value. This ELISA method can be used to determine the amount of ADAMTS 13 in plasma and other biological fluids.

### Example 12. Inhibition of Recombinant ADAMTS13 Activity by Goat anti-ADAMTS13 Antibody

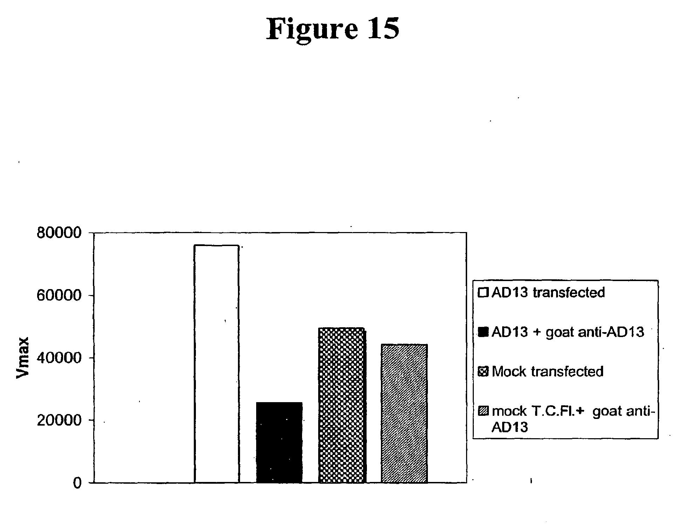

Tissue culture fluid (5 µl) from HEK 293 cells transfected with a vector coding for recombinant ADAMTS 13 or tissue culture fluid (5 µl) from mock transfected HEK 293 cells was added to 5 µg of goat anti-ADAMTS 13 antibody (Santa Cruz Biochemicals, Santa Cruz, CA) and 0.8 mM Suc-LLVY-AMC fluorescent substrate in 50 mM Tris-HCl pH 8.0 buffer (85 µl). The fluorescence (Vmax) was followed in a spectrofluorometric plate reader at Ex 360 nm/ Em 440 nm (Figure 15).

ADAMTS 13 culture fluid had more fluorescence activity than the mock transfected culture fluid. The goat anti-ADAMTS13 antibody inhibited the fluorescence from the ADAMTS 13 fluid but not from the tissue culture fluid from mock transfected cells. These results show that the fluorescence activity from the fluid from mock transfected cells is not due to ADAMTS 13.

### Example 13. Inhibition of Recombinant ADAMTS13 Activity by Acquired TTP Plasma

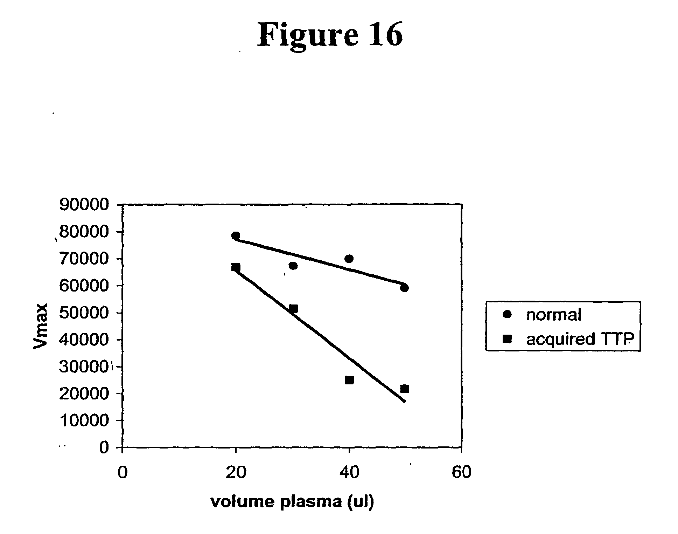

Plasma from patients with acquired TTP ("acquired TTP plasma") contains an inhibitory antibody against ADAMTS 13. Figure 16 shows that the addition of TTP plasma to tissue culture supernatant containing recADAMTS 13 inhibited the generation of a fluorescence signal when Suc-LLVY-AMC was used as a substrate. The amount of inhibition of fluorescence signal was dependent on the amount of TTP plasma added. Plasma from healthy subjects ("normal plasma") did not significantly inhibit ADAMTS13 activity, as compared to acquired TTP plasma, demonstrating the ADAMTS 13 activity was specifically inhibited by the acquired TTP plasma. These studies also show that antibodies present in acquired TTP plasmas block hydrolysis of the fluorescent Suc-LLVY-AMC substrate by recombinant ADAMTS13.

### Example 14. Inhibition of ADAMTS13 Activity on Isolated Platelets by Anti-ADAMTS13 Antibodies

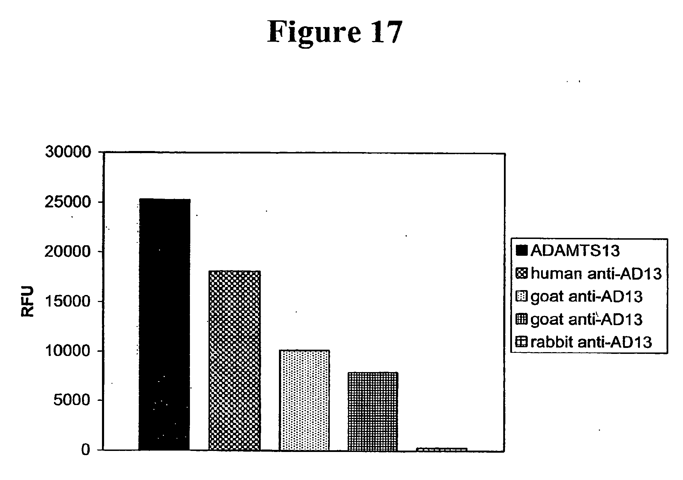

One ml of PRP was centrifuged at 10,000 rpm and the platelets in the pellet were washed with 50 mM Tris-HCl pH 8.0 buffer. The platelets were suspended in 200 µl of assay buffer. Isolated platelets (20 ul) were added to 70 µl of various anti-ADAMTS13 antibodies and incubated at room temperature for 15 minutes. The G1 and G2 goat antibodies (Santa Cruz Biochemicals, Santa Cruz, CA) were used at 200 µg/ml; the rabbit antibody against the C-terminal fragment of ADAMTS 13 (from Dr. Ginsburg) was used at 5.5 mg/ml. Purified immunoglobulin (Ig) from acquired TTP plasma was used at a concentration of 4 mg/ml. Ninety µl of 10mM Tris-HCl pH 8.0 buffer and 10 µl of 8 mM LVY-AMC were added and the fluorescence was measured over time as above. All four anti-ADAMTS 13 specific antibodies inhibited the activity of normal platelets (Figure 17). These findings indicate that the activity in platelets was due to ADAMTS 13 cleaving the LVY-AMC substrate.

### Example 15. A Method for Measuring Anti-ADAMTS13 Antibodies in Plasma Using Isolated Platelets

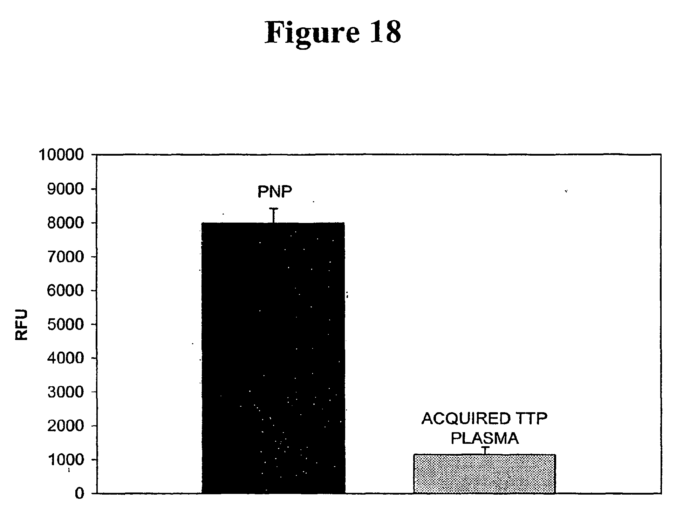

Detection of anti-ADAMTS 13 autoantibodies was performed using isolated platelets as a source of ADAMTS 13. Isolated platelets (prepared from 200 µL of normal PRP) were incubated with 200 µL pooled normal plasma (PNP) or acquired TTP plasma. The mixtures were incubated at 37°C for 30 minutes. Fifty µl were removed from each reaction mixture and added to 130 µl of 50 mM Tris-HCl pH 8.0 buffer and 20 µL of 8 mM LVY-AMC substrate. The reaction mixtures were incubated for 30 minutes at 37° C for one hour and fluorescence was monitored in a spectrofluorometric plate reader (Ex 360 nm/ Em 440 nm). The platelets incubated with normal plasma had high fluorescence signal (indicating high ADAMTS 13 activity), whereas the platelets incubated with acquired TTP plasma had low fluorescence signal (indicating low ADAMTS 13 activity) (Figure 18). These results show that anti-ADAMTS 13 autoantibodies were present in the acquired TTP plasma, but not in the normal plasma, and that autoantibodies in plasma from a patient with acquired TTP can be detected using this method. Other fluorescent ADAMTS13 substrates can be substituted for LVY-AMC. This method can be used to distinguish between congenital TTP and acquired TTP.

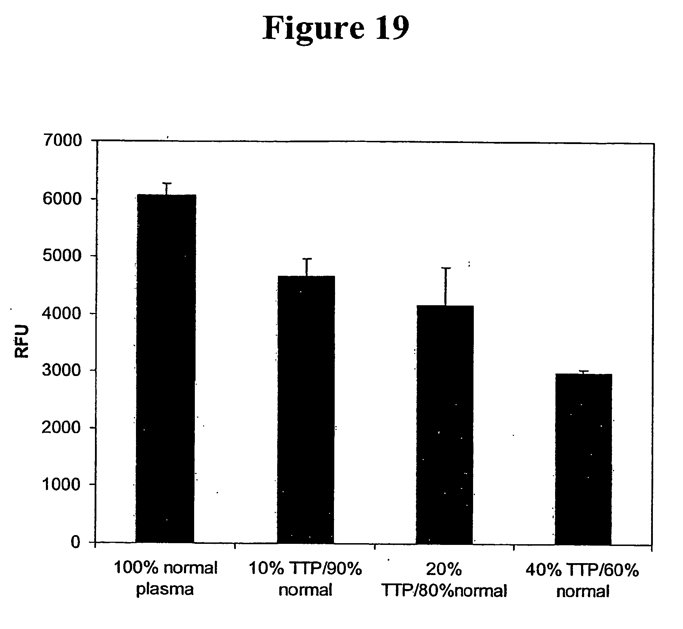

Figure 19 shows that the level of inhibition of ADAMTS 13 activity on platelets is dependent on the amount of anti-ADAMTS13 autoantibodies used in the assay, and thus the anti-ADAMTS 13 activity. Plasma from an acquired TTP patient was diluted to different amounts (10%, 20% and 40%) in normal plasma and incubated with isolated platelets in a total volume of 90 µl for 15 minutes. Ten µl of 8 mM LVY-AMC substrate was added and the fluorescence was determined after incubation at 37°C for 1 hour. The inhibition of ADAMTS 13 activity by TTP plasma was dependent upon the amount of TTP plasma used in the assay.

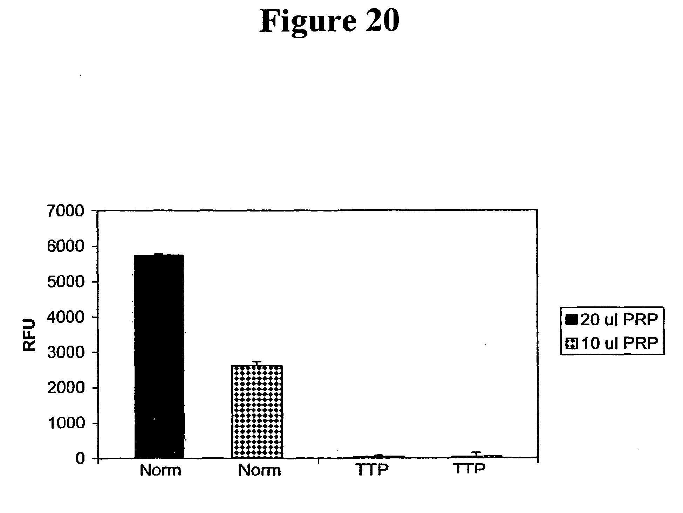

The amount of fluorescence generated over time in the assay is dependent upon the amount of platelets used in the assay. As shown in Figure 20, a higher fluorescent signal was generated in the control (normal plasma without anti-ADAMTS 13 antibodies) using more platelets. The addition of acquired TTP plasma, containing ADAMTS 13 antibodies, almost completely inhibited ADAMTS13 activity, regardless of the amount of platelets in the assay.

## Claims

1. A method for measuring ADAMTS13 activity on the surface of platelets comprising:
a) incubating a sample comprising ADAMTS13 with a substrate, wherein the sample is washed platelets, wherein the substrate comprises a peptide moiety and a chromogenic moiety, wherein the peptide moiety comprises X-Val-Tyr, X-Leu-Tyr or X-De-Tyr, wherein X is any amino acid, wherein the peptide length is 3 to 20 amino acids in length, with the proviso that the chromogenic moiety is not para-nitroniline (pNA); and
b) measuring optical density of the sample;
thereby measuring ADAMTS 13 activity on the surface of platelets.

2. The method of claim 1, wherein X is Leu.

3. The method of claim 1, wherein the peptide moiety is Leu-Val-Tyr.

4. The method of claim 1, wherein the peptide moiety is Leu-Leu-Val-Tyr.

5. The method of claim 1, wherein the peptide moiety is Suc-Leu-Z.eu-Val-Tyr.

6. The method of claim 1, wherein the chromogenic moiety is selected from the group consisting of s-benzyl, 5-amino-2-nitrobenzoic acid and 6-amino-1-naphthalenesulfonamides.

7. A method for identifying an inhibitor of ADAMTS 13 on the surface of platelets comprising:
a) incubating a sample comprising ADAMTS13 on the surface of platelets with a candidate inhibitor of ADAPTS13, wherein the sample is washed platelets; and
b) measuring ADAMTS13 activity in the test sample according to the method of claim 1;
wherein the inhibitor is identified by reduced ADAMTS 13 activity on the surface of platelets in the sample compared with ADAMTS13 activity on the surface of platelets in a control sample comprising ADAMTS13 on the surface of platelets, wherein the control sample is washed platelets, and wherein the control sample was not incubated with the candidate inhibitor.

8. The method of claim 7, wherein X is Leu.

9. The method of claim 7, wherein the peptide moiety is Leu-Val-Tyr.

10. The method of claim 7, wherein the peptide moiety is Leu-Leu-Val-Tyr.

11. The method of claim 7, wherein the peptide moiety is Suc-Leu-Leu-Val-Tyr.

12. The method of claim 7, wherein the chromogenic moiety is selected from the group consisting of s-benzyl, 5-amino-2-nitrobenzoic acid and 6-amino-1-naphthalenesulfonamides.

13. The method of claim 7, wherein the candidate inhibitor is from a chemical compound library, a phage display library, a natural chemical library or a combinatorial chemistry library.

14. The method of claim 7, wherein the inhibitor is an antibody.

15. Use of the method of claim 1 for diagnosing Thrombotic Thrombocytopenia Purpura (TTP) in a subject,
wherein ADAMTS 13 activity on the surface of platelets is measured in a test sample from the subject according to the method of claim 1, wherein the test sample is washed platelets; and wherein the method further comprises
c) comparing the ADAMTS 13 activity on the surface of platelets in the test sample to ADAMTS 13 activity on the surface of platelets in a control sample having normal ADAMTS13 activity on the surface of platelets, wherein the control sample is washed platelets;
wherein TTP is diagnosed by reduced ADAMTS 13 activity on the surface of platelets in the test sample compared with the control sample.

16. The use of claim 15, wherein X is Leu.

17. The use of claim 15, wherein the peptide moiety is Leu-Val-Tyr.

18. The use of claim 15, wherein the peptide moiety is Leu-Leu-Val-Tyr.

19. The use of claim 15, wherein the peptide moiety is Suc-Leu-Leu-Val-Tyr.

20. The use of claim 15, wherein the chromogenic moiety is selected from the group consisting of s-benzyl, 5-amino-2-nitrobenzoic acid and 6-amino-1-naphthalenesulfonamides.

21. The use of claim 15, wherein the TTP is congenital.

22. The use of claim 15, wherein the TTP is acquired.

23. Use of the method of claim 1 for diagnosing acquired TTP in a subject,
wherein a sample comprising ADAMTS13 on the surface of platelets is incubated with plasma from the subject, wherein the sample is washed platelets; and
wherein ADAPTS13 activity on the surface of platelets is measured in the sample according to the method of claim 1;
wherein acquired TTP is diagnosed by reduced ADAMTS 13 activity on the surface of platelets in the sample compared with ADAMTS13 activity on the surface of platelets in a control sample having normal ADAMTS13 activity on the surface of platelets.

24. The use of claim 23, wherein X is Leu.

25. The use of claim 23, wherein the peptide moiety is Leu-Val-Tyr.

26. The use of claim 23, wherein the peptide moiety is Leu-Leu-Val-Tyr.

27. The use of claim 23, wherein the peptide moiety is Suo-Leu-Leu-Val-Tyr.

28. The use of claim 23, wherein the chromogenic moiety is selected from the group consisting of s-benzyl, 5-amino-2-nitrobenzoic acid and 6-amino-1-naphthalenesulfonamides.

29. Use of the method of claim 1 for monitoring treatment of a patient with TTP, wherein ADAMTS 13 activity on the surface of platelets is measured according to the method of claim 1 during treatment of the patient

30. The use of claim 29, wherein the treatment is plasmaphoresis.

31. The use of claim 30, wherein the patient has acquired TTP and wherein the plasmaphoresis removes ADAMTS13 inhibitors from the patient.

32. The method of claim 1, further comprising adding coagulation Factor XIa to the sample.

## Patentansprüche

1. Verfahren zum Messen von ADAMTS13-Aktivität auf der Oberfläche von Blutplättchen, umfassend:
a) Inkubieren einer Probe, umfassend ADAMTS13, mit einem Substrat, wobei die Probe gewaschene Blutplättchen ist, wobei das Substrat eine Peptidgruppe und eine chromogene Gruppe umfasst, wobei die Peptidgruppe X-Val-Tyr, X-Leu-Tyr oder X-Ile-Tyr umfasst, wobei X eine beliebige Aminosäure ist, wobei die Peptidlänge 3 bis 20 Aminosäuren beträgt, unter der Bedingung, dass die chromogene Gruppe nicht para-Nitroanilin ist; und
b) Messen der optischen Dichte der Probe, wodurch ADAMTS 13-Aktivität auf der Oberfläche von Blutplättchen gemessen wird.

2. Verfahren nach Anspruch 1, wobei X Leu ist.

3. Verfahren nach Anspruch 1, wobei die Peptidgruppe Leu-Val-Tyr ist.

4. Verfahren nach Anspruch 1, wobei die Peptidgruppe Leu-Leu-Val-Tyr ist.

5. Verfahren nach Anspruch 1, wobei die Peptidgruppe Suc-Leu-Leu-Val-Tyr ist.

6. Verfahren nach Anspruch 1, wobei die chromogene Gruppe ausgewählt ist aus der Gruppe, bestehend aus s-Benzyl, 5-Amino-2-nitrobenzoesäure und 6-Amino-1-Naphthalensulfonamide.

7. Verfahren zum Identifizieren eines Inhibitors von ADAMTS 13 auf der Oberfläche von Blutplättchen, umfassend:
a) Inkubieren einer Probe, umfassend ADAMTS 13 auf der Oberfläche von Blutplättchen, mit einem Kandidateninhibitor von ADAMTS13, wobei die Probe gewaschene Blutplättchen ist; und
b) Messen von ADAMTS 13-Aktivität in der Testprobe gemäß dem Verfahren nach Anspruch 1;
wobei der Inhibitor identifiziert wird anhand einer verringerten ADAMTS 13-Aktivität auf der Oberfläche von Blutplättchen in der Probe im Vergleich mit ADAMTS13-Aktivität auf der Oberfläche von Blutplättchen in einer Kontrollprobe, umfassend ADAMTS 13 auf der Oberfläche von Blutplättchen, wobei die Kontrollprobe gewaschene Blutplättchen ist, und wobei die Kontrollprobe nicht mit dem Kandidateninhibitor inkubiert wurde.

8. Verfahren nach Anspruch 7, wobei X Leu ist.

9. Verfahren nach Anspruch 7, wobei die Peptidgruppe Leu-Val-Tyr ist.

10. Verfahren nach Anspruch 7,wobei die Peptidgruppe Leu-Leu-Val-Tyr ist.

11. Verfahren nach Anspruch 7, wobei die Peptidgruppe Suc-Leu-Leu-Val-Tyr ist.

12. Verfahren nach Anspruch 7, wobei die chromogene Gruppe ausgewählt ist aus der Gruppe, bestehend aus s-Benzyl, 5-Amino-2-nitrobenzoesäure und 6-Amino-1-naphthalensulfonamide.

13. Verfahren nach Anspruch 7, wobei der Kandidateninhibitor aus einer chemischen Verbindungsbibliothek, einer Phagen-Display-Bibliothek, einer Bibliothek natürlicher Chemikalien oder einer kombinatorischen Chemie-Bibliothek stammt.

14. Verfahren nach Anspruch 7, wobei der Inhibitor ein Antikörper ist.

15. Verwendung des Verfahrens nach Anspruch 1 zum Diagnostizieren von thrombotischer Thrombozytopenie-Purpura (TTP) in einem Patienten, wobei ADAMTS 13-Aktivität auf der Oberfläche von Blutplättchen in einer Testprobe aus dem Patienten gemäß dem Verfahren nach Anspruch 1 gemessen wird, wobei die Testprobe gewaschene Blutplättchen ist, und wobei das Verfahren weiterhin umfasst
c) Vergleichen der ADAMTS 13-Aktivität auf der Oberfläche von Blutplättchen in der Testprobe mit ADAMTS 13-Aktivität auf der Oberfläche von Blutplättchen in einer Kontrollprobe mit normaler ADAMTS 13-Aktivität auf der Oberfläche von Blutplättchen, wobei die Kontrollprobe gewaschene Blutplättchen ist;
wobei TTP diagnostiziert wird anhand einer verringerten ADAMTS13-Aktivität auf der Oberfläche von Blutplättchen in der Testprobe im Vergleich mit der Kontrollprobe.

16. Verwendung nach Anspruch 15, wobei X Leu ist

17. Verwendung nach Anspruch 15, wobei die Peptidgruppe Leu-Val-Tyr ist.

18. Verwendung nach Anspruch 15, wobei die Peptidgruppe Leu-Leu-Val-Tyr ist.

19. Verwendung nach Anspruch 15, wobei die Peptidgruppe Suc-Leu-Leu-Val-Tyr ist.

20. Verwendung nach Anspruch 15, wobei die chromogene Gruppe ausgewählt ist aus der Gruppe, bestehend aus s-Benzyl, 5-Amino-2-nitrobenzoesäure und 6-Amino-1-Naphthalensulfonamide.

21. Verwendung nach Anspruch 15, wobei die TTP kongenital ist.

22. Verwendung nach Anspruch 15, wobei die TTP erworben ist.

23. Verwendung des Verfahrens nach Anspruch 1 zum Diagnostizieren von erworbener TTP in einem Patienten, wobei eine Probe, umfassend ADAMTS 13 auf der Oberfläche von Blutplättchen, mit Plasma aus dem Patienten inkubiert wird, wobei die Probe gewaschene Blutplättchen ist; und
wobei ADAMTS 13-Aktivität auf der Oberfläche von Blutplättchen in der Probe gemäß dem Verfahren nach Anspruch 1 gemessen wird;
wobei erworbene TTP diagnostiziert wird anhand verringerter ADAMTS 13-Aktivität auf der Oberfläche von Blutplättchen in der Probe im Vergleich mit ADAMTS13-Aktivität auf der Oberfläche von Blutplättchen in einer Kontrollprobe mit normaler ADAMTS 13-Aktivität auf der Oberfläche von Blutplättchen.

24. Verwendung nach Anspruch 23, wobei X Leu ist

25. Verwendung nach Anspruch 23, wobei die Peptidgruppe Leu-Val-Tyr ist.

26. Verwendung nach Anspruch 23, wobei die Peptidgruppe Leu-Leu-Val Tyr ist.

27. Verwendung nach Anspruch 23, wobei die Peptidgruppe Suc-Leu-Leu-Val-Tyr ist.

28. Verwendung nach Anspruch 23, wobei die chromogene Gruppe ausgewählt ist aus der Gruppe, bestehend aus s-Benzyl, 5-Amino-2-nitrobenzoesäure und 6-Amino-1-Naphthalensulfonamide.

29. Verwendung des Verfahrens nach Anspruch 1 zum Überwachen einer Behandlung eines Patienten mit TTP, wobei ADAMTS 13-Aktivität auf der Oberfläche von Blutplättchen gemäß dem Verfahren nach Anspruch 1 während der Behandlung des Patienten gemessen wird.

30. Verwendung nach Anspruch 29, wobei die Behandlung Plasmaphorese ist.

31. Verwendung nach Anspruch 30, wobei der Patient erworbene TTP hat, und wobei die Plasmaphorese ADAMTS 13-Inhibitoren von dem Patienten entfernt.

32. Verfahren nach Anspruch 1, weiterhin umfassend die Zugabe von Koagulationsfaktor XIa zu der Probe.

## Revendications

1. Procédé de mesure de l'activité de l'ADAMTS13 sur la surface des plaquettes comprenant :
a) l'incubation d'un échantillon comprenant l'ADAMTS13 avec un substrat, dans lequel l'échantillon est constitué de plaquettes lavées, dans lequel le substrat comprend un fragment peptidique et un fragment chromogène, dans lequel le fragment peptidique comprend X-Val-Tyr, X-Leu-Tyr ou X-Ile-Tyr, dans lequel X est un acide aminé quelconque, dans lequel la longueur de peptide est de 3 à 20 acides aminés en longueur, sous réserve que le fragment chromogène n'est pas la para-nitroaniline (pNA) ; et
b) la mesure de la densité optique de l'échantillon ;
mesurant ainsi l'activité de l'ADAMTS13 sur la surface des plaquettes.

2. Procédé selon la revendication 1, dans lequel X est Leu.

3. Procédé selon la revendication 1, dans lequel le fragment peptidique est Leu-Val-Tyr.

4. Procédé selon la revendication 1, dans lequel le fragment peptidique est Leu-Leu-Val-Tyr.

5. Procédé selon la revendication 1, dans lequel le fragment peptidique est Suc-Leu-Leu-Val-Tyr.

6. Procédé selon la revendication 1, dans lequel le fragment chromogène est choisi parmi le groupe consistant en le s-benzyle, l'acide 5-amino-2-nitrobenzoïque et des 6-amino-1-naphtalènesulfonamides.

7. Procédé d'identification d'un inhibiteur de l'ADAMTS13 sur la surface des plaquettes comprenant :
a) l'incubation d'un échantillon contenant l'ADAMTS13 sur la surface des plaquettes avec un inhibiteur candidat de l'ADAMTS13, dans lequel l'échantillon est constitué de plaquettes lavées ; et
b) la mesure de l'activité de l'ADAMTS13 dans l'échantillon de test conformément au procédé selon la revendication 1 ;
dans lequel l'inhibiteur est identifié par une activité réduite de l'ADAMTS13 sur la surface des plaquettes dans l'échantillon par rapport à l'activité de l'ADAMTS13 sur la surface des plaquettes dans un échantillon témoin comprenant l'ADAMTS13 sur la surface des plaquettes, dans lequel l'échantillon témoin est constitué de plaquettes lavées, et dans lequel l'échantillon témoin n'a pas été incubé avec l'inhibiteur candidat.

8. Procédé selon la revendication 7, dans lequel X est Leu.

9. Procédé selon la revendication 7, dans lequel le fragment peptidique est Leu-Val-Tyr.

10. Procédé selon la revendication 7, dans lequel le fragment peptidique est Leu-Leu-Val-Tyr.

11. Procédé selon la revendication 7, dans lequel le fragment peptidique est Suc-Leu-Leu-Val-Tyr.

12. Procédé selon la revendication 7, dans lequel le fragment chromogène est choisi parmi le groupe consistant en le s-benzyle, l'acide 5-amino-2-nitrobenzoïque et des 6-amino-1-naphtalènesulfonamides.

13. Procédé selon la revendication 7, dans lequel l'inhibiteur candidat provient d'une banque de composés chimiques, d'une banque d'expression phagique, d'une banque de produits chimiques naturels ou d'une banque de chimie combinatoire.

14. Procédé selon la revendication 7, dans lequel l'inhibiteur est un anticorps.

15. Utilisation du procédé selon la revendication 1 pour diagnostiquer un purpura thrombotique thrombocytopénique (PTT) chez un sujet,
dans laquelle l'activité de l'ADAMTS13 sur la surface des plaquettes est mesurée dans un échantillon de test provenant du sujet conformément au procédé selon la revendication 1, dans laquelle l'échantillon de test est constitué de plaquettes lavées ; et dans laquelle le procédé comprend en outre
c) la comparaison de l'activité de l'ADAMTS13 sur la surface des plaquettes dans l'échantillon de test à l'activité de l'ADAMTS13 sur la surface des plaquettes dans un échantillon témoin ayant une activité normale de l'ADAMTS13 sur la surface des plaquettes, dans laquelle l'échantillon témoin est constitué de plaquettes lavées ;
dans laquelle le PTT est diagnostiqué par une activité réduite de l'ADAMTS13 sur la surface des plaquettes dans l'échantillon de test par rapport à l'échantillon témoin.

16. Utilisation selon la revendication 15, dans laquelle X est Leu.

17. Utilisation selon la revendication 15, dans laquelle le fragment peptidique est Leu-Val-Tyr.

18. Utilisation selon la revendication 15, dans laquelle le fragment peptidique est Leu-Leu-Val-Tyr.

19. Utilisation selon la revendication 15, dans laquelle le fragment peptidique est Suc-Leu-Leu-Val-Tyr.

20. Utilisation selon la revendication 15, dans laquelle le fragment chromogène est choisi parmi le groupe consistant en le s-benzyle, l'acide 5-amino-2-nitrobenzoïque et des 6-amino-1-naphtalènesulfonamides.

21. Utilisation selon la revendication 15, dans laquelle le PTT est congénital.

22. Utilisation selon la revendication 15, dans laquelle le PTT est acquis.

23. Utilisation du procédé selon la revendication 1 pour diagnostiquer un PTT acquis chez un sujet,
dans laquelle un échantillon comprenant l'ADAMTS13 sur la surface des plaquettes est incubé avec du plasma provenant du sujet, dans laquelle l'échantillon est constitué de plaquettes lavées ; et
dans laquelle l'activité de l'ADAMTS13 sur la surface des plaquettes est mesurée dans l'échantillon conformément au procédé selon la revendication 1 ;
dans laquelle le PTT acquis est diagnostiqué par une activité réduite de l'ADAMTS13 sur la surface des plaquettes dans l'échantillon par rapport à l'activité de l'ADAMTS13 sur la surface des plaquettes dans un échantillon témoin ayant une activité normale de l'ADAMTS13 sur la surface des plaquettes.

24. Utilisation selon la revendication 23, dans laquelle X est Leu.

25. Utilisation selon la revendication 23, dans laquelle le fragment peptidique est Leu-Val-Tyr.

26. Utilisation selon la revendication 23, dans laquelle le fragment peptidique est Leu-Leu-Val-Tyr.

27. Utilisation selon la revendication 23, dans laquelle le fragment peptidique est Suc-Leu-Leu-Val-Tyr.

28. Utilisation selon la revendication 23, dans laquelle le fragment chromogène est choisi parmi le groupe consistant en le s-benzyle, l'acide 5-amino-2-nitrobenzoïque et des 6-amino-1-naphtalènesulfonamides.

29. Utilisation du procédé selon la revendication 1 pour surveiller le traitement d'un patient atteint d'un PTT, dans laquelle l'activité de l'ADAMTS13 sur la surface des plaquettes est mesurée conformément au procédé selon la revendication 1 pendant le traitement du patient.

30. Utilisation selon la revendication 29, dans laquelle le traitement est une plasmaphérèse.

31. Utilisation selon la revendication 30, dans laquelle le patient est atteint d'un PTT acquis et dans laquelle la plasmaphérèse élimine les inhibiteurs de l'ADAMTS13 du patient.

32. Procédé selon la revendication 1, comprenant en outre l'ajout du Facteur de coagulation XIa à l'échantillon.
